# EUROPÄISCHE PATENTSCHRIFT

(11) **EP 0 663 384 B1**
(45) Veröffentlichungstag und Bekanntmachung des Hinweises auf die Patenterteilung: **04.06.1997**
(21) Anmeldenummer: 94117975.6
(22) Anmeldetag: 15.11.1994
(51) Int. Cl.: C07C 63/313, C08G 59/50, C07C 279/02, C07C 279/04

(54) **Salze der Pyromellitsäure, ein Verfahren zu ihrer Herstellung sowie deren Verwendung**
Pyromellitic acid salts, process for preparing them and use
Sels d'acide pyroméllitique, leur procédé de préparation et leur emploi

(30) Priorität: 14.01.1994 DE 4400931
(43) Veröffentlichungstag der Anmeldung: 19.07.1995
(73) Patentinhaber: HÜLS AKTIENGESELLSCHAFT, 45764 Marl (DE)
(72) Erfinder: Gras, Rainer, Dr., D-44879 Bochum (DE); Wolf, Elmar, Dr., D-45661 Recklinghausen (DE)

(56) Entgegenhaltungen:
- DE-A- 1 900 825

## Beschreibung

Die Erfindung betrifft neue Salze der Pyromellitsäure, ein Verfahren zu ihrer Herstellung sowie deren Verwendung zur Herstellung von matten Epoxid- sowie Hybrid-Pulverbeschichtungen.

In der DE-OS 23 24 696 wird ein Verfahren zur Herstellung von matten Überzügen beschrieben, indem man Epoxidharze mit Salzen aus Pyromellitsäure und cyclischen Amidinen (Imidazolinen, Tetrahydropyrimidinen) härtet. Zur Härtung benötigt man Temperaturen von 180 °C - 200 °C.

Es besteht großes Interesse an Härtern mit den vorteilhaften Eigenschaften der Pyromellitsäuresalze der DE-OS 23 24 696 und mit denen man um ca. 20 °C tiefer härten kann, wie dies zur Zeit mit den Salzen der DE-OS 23 24 696 nicht möglich ist. Aufgabe der Erfindung war somit, Härter mit den vorteilhaften Eigenschaften der Pyromellitsäuresalze der DE-OS 23 24 696 ohne deren Nachteile aufzufinden.

Überraschenderweise konnte diese Aufgabe durch Einsatz von Salzen der Pyromellitsäure mit Guanidinen gelöst werden.

Gegenstand der vorliegenden Erfindung sind somit Salze der Pyromellitsäure, welche dadurch gekennzeichnet sind, daß sie aus 1 Mol Pyromellitsäure und 0,5 bis 2 mol Guanidinen der folgenden Zusammensetzung bestehen: 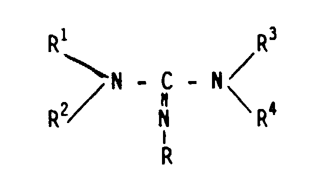 wobei R, R¹, R², R³, R⁴: gleiche oder verschiedene Reste aus der Gruppe: H, (cyclo)-Alkyl-, aromatischer KW-Rest mit 1 - 8 C-Atomen bedeuten und R¹ und R² sowie R³ und R⁴ einen gemeinsamen Ring bilden können, der ein O-Atom als Heteroatom enthalten kann.

Zur Herstellung der erfindungsgemäßen Salze der Pyromellitsäure eignen sich die Guanidine, wie z. B. Tetramethyl-guanidin, Tetramethylcyclohexylguanidin, N.N'.N''-Triphenylguanidin, N.N'-Dicyclohexyl-4-morpholin-carbonamid. Der basische N-Gehalt der erfindungsgemäßen Salze beträgt 1 - 10 mmol NH₂/g, der Carboxylgruppengehalt 3 - 13 mmol/g.

Bei den erfindungsgemäßen Verbindungen handelt es sich um farblose bis zum Teil intensiv gelb gefärbte Substanzen mit Schmelzpunkten von 140 °C bis ca. 250 °C. Ihre Herstellung erfolgt in bekannter Weise, wobei zu der in Wasser oder Ethanol gelösten Pyromellitsäure in der Siedehitze die Aminkomponente portionsweise zugegeben wird.

Nach beendeter Aminzugabe wird noch ca. 1 Stunde weiter erhitzt. Anschließend wird auf Raumtemperatur abgekühlt. Der gebildete Niederschlag, wenn in Ethanol gearbeitet wird, wird abfiltriert und im Vakuumtrockenschrank bei 60 °C getrocknet. In Wasser ist das Reaktionsprodukt löslich; das Wasser wird nach bekannten Verfahren, z. B. durch Abdestillieren, Sprühtrocknen, entfernt.

Ein weiterer Gegenstand der Erfindung ist somit ein Verfahren zur Herstellung von Salzen der Pyromellitsäure nach Anspruch 1, wobei 1 mol Pyromellitsäure mit 0,5 - 2 mol der Guanidine in H₂O bzw. Ethanol bei 50 - 100 °C umgesetzt, und nach beendeter Reaktion das Reaktionsprodukt vom Lösungsmittel befreit oder durch Sprühtrocknung isoliert wird.

Wie bereits aufgeführt, eignen sich die erfindungsgemäßen Verbindungen zur Herstellung von matten Epoxid- sowie Hybrid-Pulverbeschichtungen, wie sie z. B. in der DE-OS 23 24 696 beschrieben werden.

Ferner ist Gegenstand der Erfindung die Verwendung der Salze der Pyromellitsäure nach Anspruch 1 zur Herstellung von matten Hybrid-Pulverbeschichtungen, wobei das EP-Harz neben dem COOH enthaltenden Polyester und gegebenenfalls einem ∈-Caprolactam blockierten Isophorondiisocyanat-Addukt 0,5 - 12 Gew.-%, vorzugsweise 1 - 7 Gew.-% und insbesondere 2,0 - 5,5 Gew.-%, der Salze der Pyromellitsäure enthält.

Schließlich ist Gegenstand der Erfindung die Verwendung der Salze der Pyromellitsäure nach Anspruch 1 zur Herstellung von matten Epoxid-Pulverbeschichtungen, wobei das EP-Harz 3 - 12 Gew.-%, vorzugsweise 4 - 7 Gew.-%, der Salze der Pyromellitsäure enthält.

Die verwendeten Polyepoxide sind feste, harzartige Stoffe, die im Bereich 60 - 150 °C, vorzugsweise 70 - 110 °C schmelzen und die im Durchschnitt mehr als eine 1.2-Epoxidgruppe pro Molekül enthalten. Im Prinzip kommen alle Verbindungen infrage, die mehr als eine 1.2-Epoxidgruppe pro Molekül enthalten; bevorzugt werden allerdings handelsübliche EP-Harze, wie sie durch Umsetzung von Bisphenol A und Epichlorhydrin erhalten werden, mit einem EP-Äquivalentgewicht zwischen 400 - 3000, bevorzugt 800 - 1000, eingesetzt.

Bei den carboxylgruppenhaltigen Polymeren handelt es sich um Polyesterpolycarbonsäuren, die aus Polyolen und Polycarbonsäuren bzw. deren Derivaten hergestellt werden. Der Schmelzbereich dieser sauren Polyester liegt in einem Bereich von 60 - 160 °C, vorzugsweise 80 - 120 °C; ihre Säurezahl variiert von 10 - 150 mg KOH/g, vorzugsweise 30 - 60 mg KOH/g. Die OH-Zahlen sollen unter 10 mg KOH/g liegen.

Für die Herstellung der erfindungsgemäß zu verwendenden Polyesterpolycarbonsäuren einzusetzende Polycarbonsäuren sind z. B. Oxal-, Adipin-, 2.2.4(2.4.4)-Trimethyladipin-, Azelain-, Sebacin-, Decandicarbon-, Dodecandicarbon-, Fumar-, Phthal-, Isophthal-, Terephthal-, Trimellit-, Pyromellitsäure. Als Polyole für die sauren Polyester werden folgende verwendet: Ethylenglykol, 1.2- und 1.3-Propandiol, 1.2-, 1.3-, 1.4- und 2.3-Butandiol, 1.5-Pentandiol, 3-Methyl-1.5-Pentandiol, Neopentylglykol, 1.6-Hexandiol, 1.12-Dodecandiol, 2.2.4(2.4.4)-Trimethyl-1.6-Hexandiol, Trimethylolpropan, Glyzerin, Pentaerythrit, 1.4-Bishydroxymethyl-cyclohexan, Cyclohexan-1.4-diol, Diethylenglykol, Triethylenglykol sowie Dipropylenglykol. Selbstverständlich können auch Hydroxylgruppen enthaltende Polyester, die nach bekannten Verfahren aus Polycarbonsäuren und Polyolen hergestellt werden, mit Polycarbonsäuren und/oder Polycarbonsäure-anhydriden zu den Polyester-polycarbonsäuren umgesetzt werden.

Die Mengen der einzelnen Pulverlackbindemittelkomponenten können weitgehend variiert werden.

Im Falle der ausschließlichen Verwendung der handelsüblichen EP-Harze auf Bisphenol A-Basis (plus Epichlorhydrin) beträgt die Härterkonzentration 3 - 12 Gew.-%. Im Falle der Verwendung von Gemischen aus Epoxidharzen des Typs Diglycidylester des Bisphenol A und Carboxylgruppen enthaltender Polyester richtet sich das Mengenverhältnis nach der Säurezahl des Carboxylpolyesters. So wird z. B. üblicherweise bei einer Säurezahl 30 - 50 mg KOH/g das Gewichtsverhältnis EP-Harz/Carboxylpolyester 60 : 40 bis 80 : 20, vorzugsweise 70 : 30, betragen. Die Konzentration der erfindungsgemäßen Salze beträgt in diesen EP-Harz/Carboxylpolyester-Gemischen 2 - 10 Gew.-%. Zur Herstellung des Pulverlacks werden die Bindemittel zusammen mit dem Verlaufmittel, Pigment und den UV- wie Oxidationsstabilisatoren zunächst gemischt und in einem Extruder bei 80 - 130 °C homogenisiert. Das Extrudat wird nach Abkühlung auf Raumtemperatur zu einem Pulverlack gemahlen, wobei die mittlere Teilchengröße ca. 40 - 80 µm, vorzugsweise 50 µm, betragen sollte.

Das Auftragen der so hergestellten Pulverlacke auf geeignete Substrate kann nach den bekannten Verfahren, wie z. B. durch elektrostatisches Pulversprühen, Wirbelsintern erfolgen. Nach dem Auftragen des Pulverlacks nach einem der genannten Verfahren werden die beschichteten Substrate zur Aushärtung auf Temperaturen von 150 - 220 °C innerhalb von 30 - 6 min erhitzt. Die so hergestellten Lackfilme zeichnen sich durch sehr guten Verlauf und eine hervorragende Lösemittelbeständigkeit und eine matte Oberfläche aus, wobei der Glanzgrad in einem weiten Bereich beliebig einstellbar ist.

### I. Herstellung der erfindungsgemäßen Salze

### Allgemeine Herstellungsvorschrift

Die in der folgenden Tabelle aufgeführten Salze der Pyromellitsäure wurden in folgender Weise hergestellt: Zu der in Wasser gelösten Pyromellitsäure wurde das Amin zugetropft. Nach beendeter Aminzugabe wurde noch ca. 1 h weiter erhitzt (bei 60 - 80 °C) und anschließend das Wasser abdestilliert. Zur vollständigen Entfernung des Wassers wurde im Vakuumtrockenschrank bei 80 °C getrocknet.

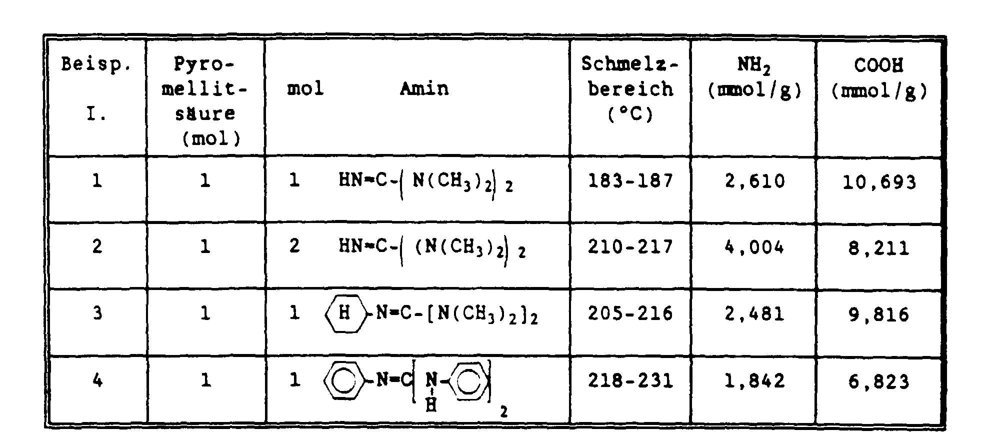

### II. Epoxidharz

In den Anwendungsbeispielen wurde als Epoxidharzverbindung eins auf Bisphenol A-Basis eingesetzt. Es ist durch folgende Kenndaten charakterisiert:

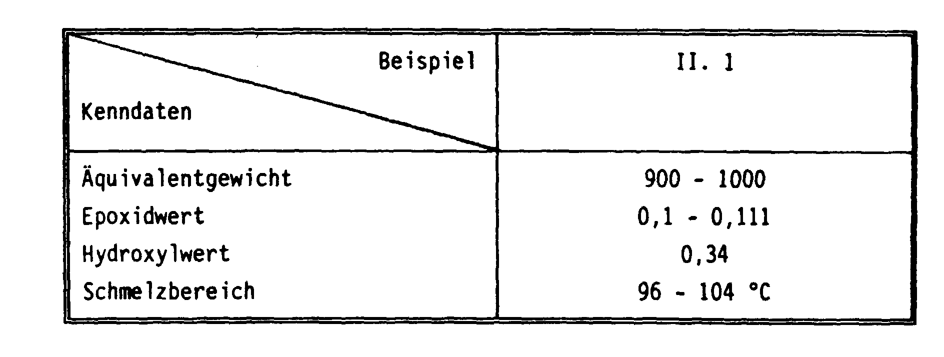

### III. Epoxidharz-Pulverlacke

Zur Herstellung der Pulverlacke wurden die gemahlenen Produkte - Härter, Epoxidharz und Verlaufmittel-Masterbatch¹ - mit dem Weißpigment (TiO₂) in einem Kollergang innig vermischt und anschließend im Extruder bei 90 bis 110 °C homogenisiert. Nach dem Erkalten wurde das Extrudat gebrochen und in einer Stiftmühle auf eine Korngröße < 100 µm gemahlen. Das so hergestellte Pulver wurde mit einer elektrostatischen Pulverspritzanlage bei 60 kV auf entfettete, gegebenenfalls vorbehandelte Stahlbleche appliziert und in einem Labor -Umlufttrockenschrank eingebrannt.
¹ Verlaufmittel Masterbatch 10 Gew.-% Verlaufmittel auf Basis von polymeren Butylacrylaten werden mit den Epoxidharzen in der Schmelze homogenisiert und nach dem Erstarren zerkleinert.

Die Abkürzungen in den folgenden Tabelle bedeuten:

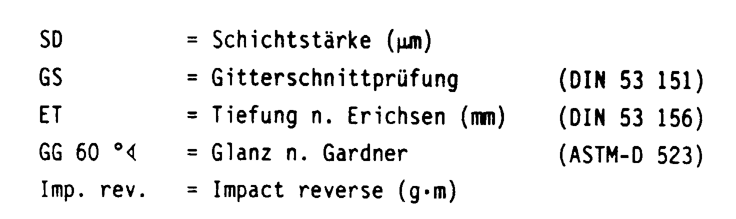

Die Berechnung der Lack-Formulierungen erfolgte nach folgendem Schema:

| | |
|---|---|
| | Gew.-% EP = Epoxid |
| B - V = EP | B = Gew.-% Bindemittel |
| | V = Gew.-% Vernetzer |
| B = 100 - Z | Z = Gew.-% Zuschlagstoffe [40 Gew.-% Weißpigment (TiO₂), 0,5 Gew.-% Verlaufmittel] |

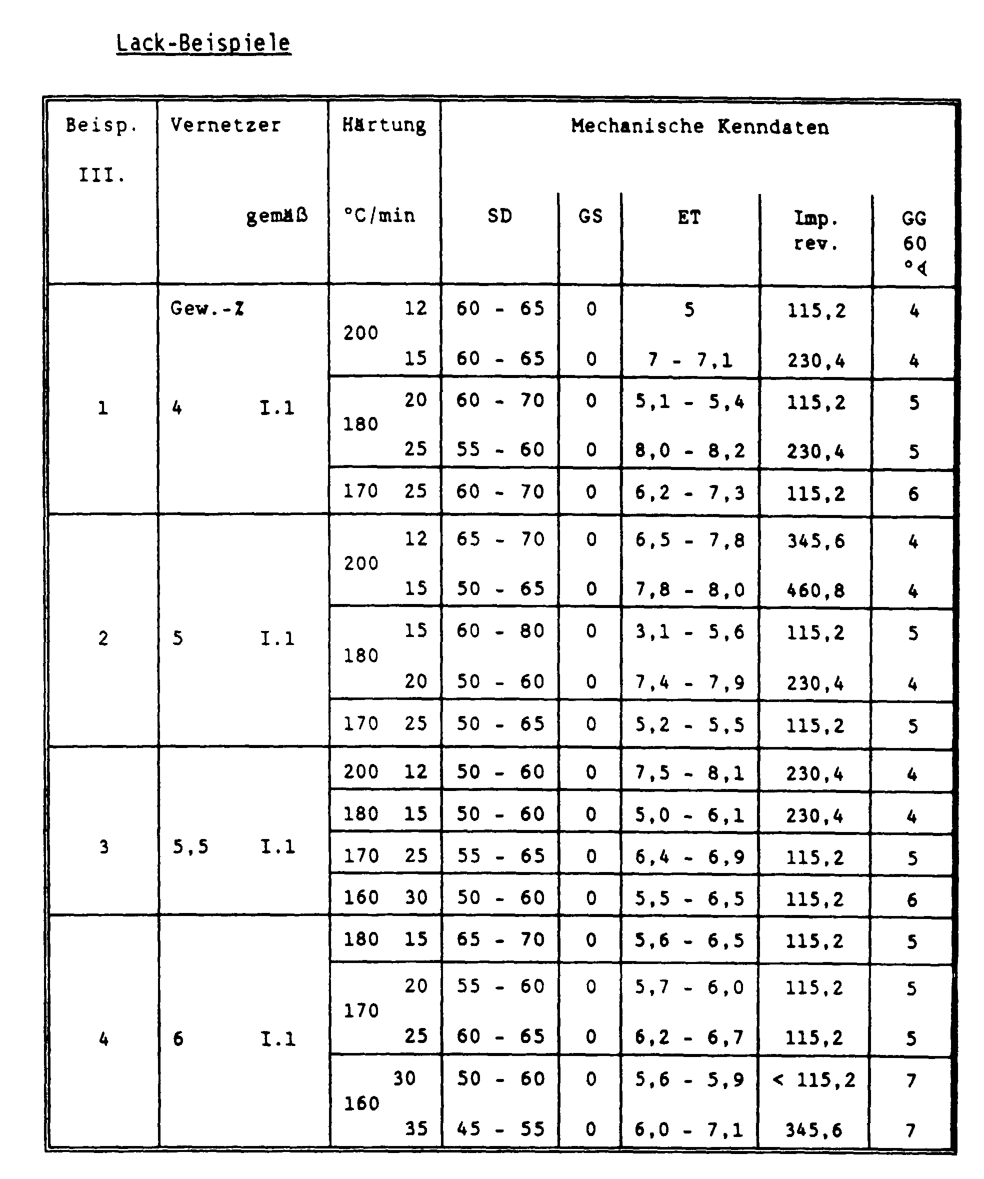

### IV. Carboxylgruppenhaltiger Polyester

Zur Herstellung von Hybrid-Pulverlacken wurden die nachfolgend beschriebenen carboxylgruppenhaltigen Polyester mit folgenden Kenndaten

| | I | II |
|---|---|---|
| Säurezahl: | 52 - 58 mg KOH/g | 36 mg KOH/g |
| Schmelzbereich: | 104 - 106 °C | 91 - 94 °C |
| Glasumwand lungstemperatur: | ca. 58 °C | 64 °C |
| Viskosität bei 160 °C: | 33.400 mPa·s | 58.000 mPa·s |

### V. Hybrid-Pulverlacke

Die Aufarbeitung der Rohstoffe sowie die Herstellung und Applikation erfolgt analog III.

Beispiel 1

Nach dem beschriebenen Verfahren wurde der Pulverlack mit folgender Rezeptur hergestellt, appliziert und zwischen 170 °C und 200 °C eingebrannt.

| | |
|---|---|
| 390,0 Gew.-T. | Epoxid gemäß II.1 |
| 75,0 Gew.-T. | Vernetzer gemäß I.1 |
| 400,0 Gew.-T. | Weißpigment (TiO₂) |
| 50,0 Gew.-T. | Verlaufmittel-Masterbatch |
| 75,0 Gew.-T. | Polyester gemäß IV.1 |

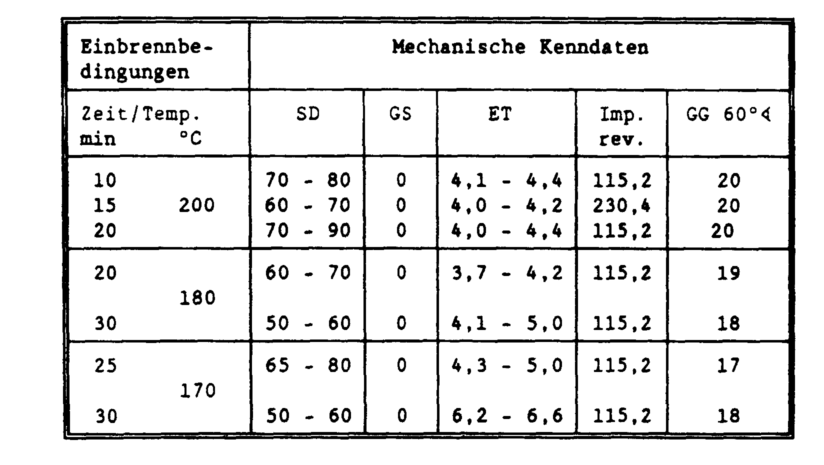

### Beispiel 2

Nach dem beschriebenen Verfahren wurde der Pulverlack mit folgender Rezeptur hergestellt, appliziert und zwischen 180 °C und 200 °C eingebrannt.

| | |
|---|---|
| 350,0 Gew.-T. | Epoxid gemäß II.1 |
| 55,0 Gew.-T. | Vernetzer gemäß I.1 |
| 400,0 Gew.-T. | Weißpigment (TiO₂) |
| 50,0 Gew.-T. | Verlaufmittel-Masterbatch |
| 145,0 Gew.-T. | Polyester gemäß IV.1 |

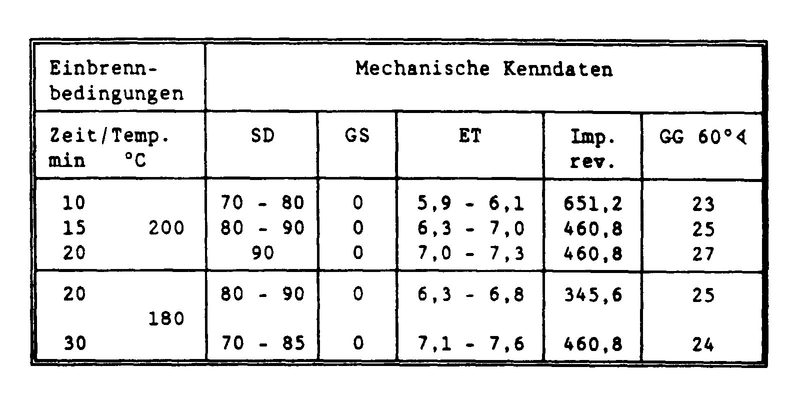

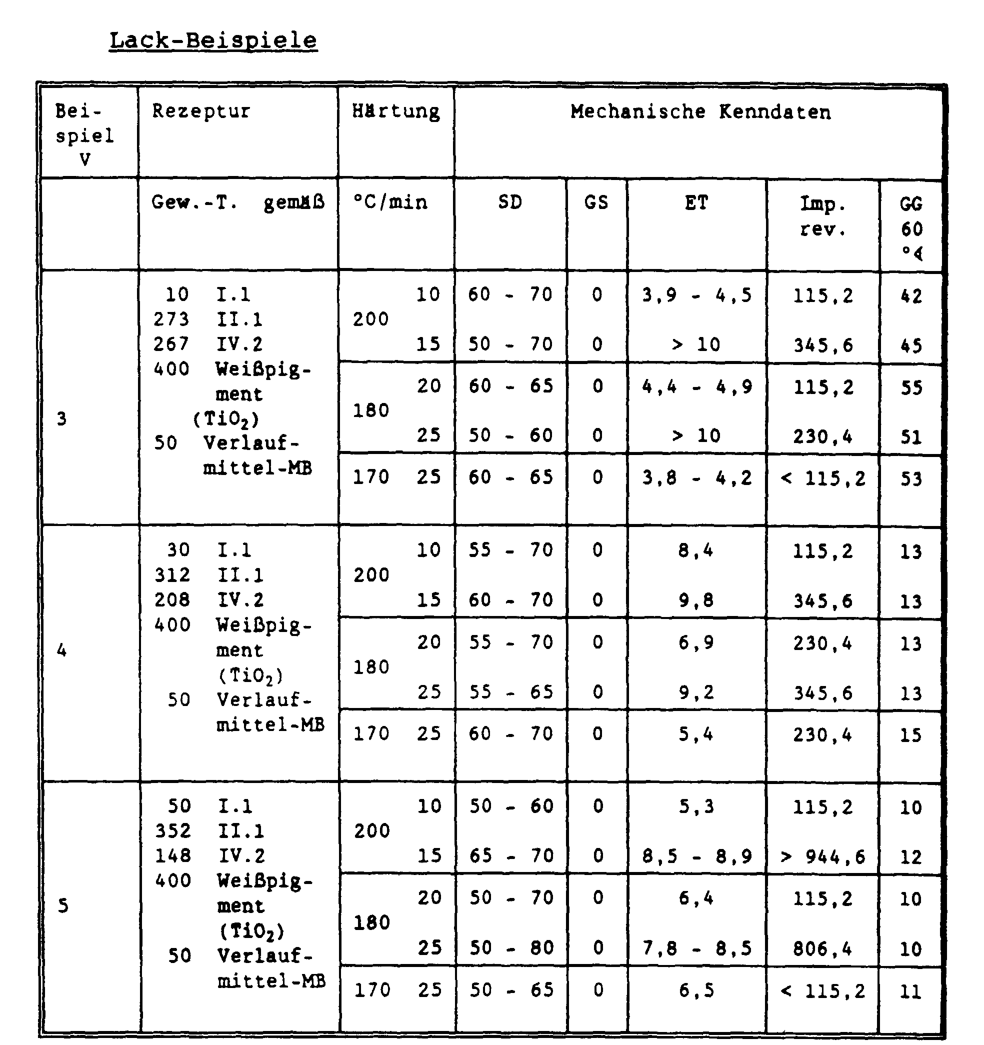

## Patentansprüche

1. Salze der Pyromellitsäure,
dadurch gekennzeichnet,
daß sie aus 1 Mol Pyromellitsäure und 0,5 - 2 mol Guanidinen der folgenden Zusammensetzung bestehen: 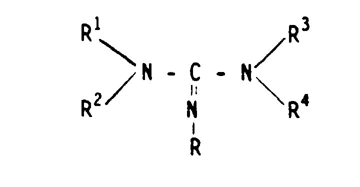 wobei R, R¹, R², R³, R⁴: gleiche oder verschiedene Reste aus der Gruppe: H, (cyclo)-Alkyl-, aromatischer KW-Rest mit 1 - 8 C-Atomen bedeuten und R¹ und R² sowie R³ und R⁴ einen gemeinsamen Ring bilden können, der ein 0-Atom als Heteroatom enthalten kann.

2. Verfahren zur Herstellung von Salzen der Pyromellitsäure nach Anspruch 1,
dadurch gekennzeichnet,
daß 1 mol Pyromellitsäure mit 0,5 - 2 mol der Guanidine in H₂O bzw. Ethanol bei 50 - 100 °C umgesetzt, und nach beendeter Reaktion das Reaktionsprodukt vom Lösungsmittel nach bekannten Verfahren befreit wird.

3. Verwendung der Salze der Pyromellitsäure nach den Ansprüchen 1 und 2 zur Herstellung von matten Epoxid-Pulverbeschichtungen,
dadurch gekennzeichnet,
daß das EP-Harz 3 - 12 Gew.-%, vorzugsweise 4 - 7 Gew.-%, der Salze der Pyromellitsäure enthält.

4. Verwendung der Salze der Pyromellitsäure nach Anspruch 1 zur Herstellung von matten Hybrid-Pulverbeschichtungen,
dadurch gekennzeichnet,
daß das EP-Harz neben dem COOH enthaltenden Polyester und gegebenenfalls einem ∈-Caprolactam blockierten Isophorondiisocyanat-Addukt 0,5 - 12 Gew.-%, vorzugsweise 1 - 7 Gew.-% und insbesondere 2,0 - 5,5 Gew.-%, der Salze der Pyromellitsäure enthält.

## Claims

1. A salt of pyromellitic acid, characterized in that it comprises 1 mol of pyromellitic acid and 0.5 - 2 mol of guanidines of the following composition: 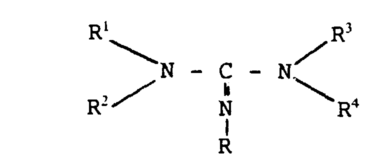 in which R, R¹, R², R³ and R⁴ are identical or different radicals from the group consisting of hydrogen (cyclo)alkyl and aromatic hydrocarbon residues having 1 - 8 carbon atoms, and R¹ and R² and also R³ and R⁴ may jointly form a ring, which may contain an oxygen atom as heteroatom.

2. A process for the preparation of a salt of pyromellitic acid according to claim 1, characterized in that I mol of pyromellitic acid is reacted with 0.5 - 2 mol of the guanidines in H₂O and/or ethanol at 50 - 100°C and, after reaction has finished, the reaction product is freed from the solvent by known methods.

3. Use of the salt of pyromellitic acid according to either of claims 1 and 2 for the production of matt epoxy powder coatings, characterized in that the epoxy resin contains 3 - 12% by weight, preferably 4 - 7% by weight, of the salt of pyromellitic acid.

4. Use of the salt of pyromellitic acid according to claim 1 for the production of matt hybrid powder coatings, characterized in that the epoxy resin contains 0.5 - 12% by weight, preferably 1 - 7% by weight and in particular 2.0 - 5.5% by weight, of the salt of pyromellitic acid, in addition to the COOH-containing polyester and, if desired, an isophorone diisocyanate adduct blocked with ∈-caprolactam.

## Revendications

1. Sels de l'acide pyromellitique,
caractérisés en ce qu'
ils sont constitués d'une mole d'acide pyromellitique et de 0,5 à 2 moles de guanidine de composition suivante : 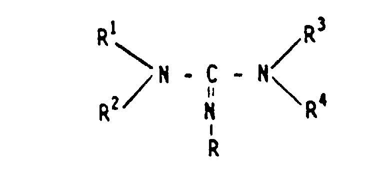 dans laquelle R, R¹, R², R³, R signifient des radicaux identiques ou différents, choisis dans le groupe de H, (cyclo)-alkyl, radical hydrocarbure aromatique ayant de 1 à 8 atomes de carbone et R¹ et R² ainsi que R³ et R⁴ peuvent former un cycle commun qui peut renfermer un atome d'O en tant qu'hétéroatome.

2. Procédé d'obtention de sels d'acide pyromellitique selon la revendication 1,
caractérisé en ce que
l'on fait réagir 1 mole d'acide pyromellitique avec 0,5 à 2 mole de guanidine dans de l'eau ou l'éthanol à 50-100°C et après achèvement de la réaction on débarrasse le produit réactionnel de solvant selon les procédés connus.

3. Utilisation des sels de l'acide pyromellitique selon les revendications 1 et 2 pour la fabrication d'enductions pulvérulentes mates par un époxyde,
caractérisée en ce que
la résine EP renferme 3-12 % en poids, de préférence 4 à 7 % en poids, de sels d'acide pyromellitique.

4. Utilisation des sels d'acide pyromellitique selon la revendication 1 pour la fabrication d'enductions mates en poudre hybride,
caractérisée en ce que
la résine EP renferme les sels d'acide pyromellitique à côté du polyester contenant COOH et le cas échéant un adduit d'isophorone diisocyanate bloqué par un ε-caprolactame, 0,5-12 % en poids, de préférence 1 à 7 % en poids et en particulier 2,0-5,5 % en poids.
